# EUROPÄISCHE PATENTSCHRIFT

(11) **EP 2 874 031 B1**
(45) Veröffentlichungstag und Bekanntmachung des Hinweises auf die Patenterteilung: **16.08.2017**
(21) Anmeldenummer: 14189976.5
(22) Anmeldetag: 23.10.2014
(51) Int. Cl.: G05B 19/042, A47J 43/07, G05B 15/02

(54) **Anordnung mit einer Küchenmaschine und einem Computersystem**
System comprising an automated food processor and a computer system
Système informatique avec un robot ménager

(30) Priorität: 08.11.2013 DE 102013112310
(43) Veröffentlichungstag der Anmeldung: 20.05.2015
(73) Patentinhaber: Vorwerk & Co. Interholding GmbH, 42275 Wuppertal (DE)
(72) Erfinder: Haase, Corinna, 8038 Zürich (CH); Resende, Maria Jose, 8832 Wollerau (CH); Wehlig, Ramona, 8810 Horgen (CH); Ganns, Julius, 42119 Wuppertal (DE); Hilgers, Stefan, 45357 Essen (DE)
(74) Vertreter: Müller, Enno

(56) Entgegenhaltungen:
- DE-A1-102007 031 372
- DE-A1-102009 058 258
- DE-A1-102010 060 650

## Beschreibung

Die Erfindung betrifft eine Anordnung, mit einem Computersystem und einer Küchenmaschine, wobei das Computersystem eine Datenverarbeitungseinrichtung aufweist, und wobei die Küchenmaschine einen Mikroprozessor, ein aufheizbares Rührgefäß und ein in dem Rührgefäß befindliches Rührwerk aufweist, wobei der Mikroprozessor eingerichtet ist, anhand eines erstellten Steuerprogramms das Rührwerk und/oder die Heizung zu steuern.

Aus dem Stand der Technik sind Küchenmaschinen in vielfachen Ausgestaltungen bekannt und dienen insbesondere im Haushaltsbereich der Zubereitung von Speisen. Oftmals weisen die Küchenmaschinen ein Rührgefäß mit einem elektromotorisch angetriebenen Rührwerk auf, wobei der Antrieb des Rührwerks in der Küchenmaschine vorgesehen ist. Zudem weisen derartige Küchenmaschinen, so beispielsweise die unter der Handelsbezeichnung "Vorwerk-Thermomix" bekannte Küchenmaschine, weitere Einrichtungen zur Zubereitung der Speisen, auch Gargut genannt, auf, so unter anderem eine Heizund/ oder Gareinrichtung, eine Waage, unterschiedlichste Ausgestaltungen von Rührwerken, wie Schaber etc. Zur Bedienung vorgenannter Einrichtungen sind an der Küchenmaschine neben einem Display verschiedene Tasten und Schalter vorgesehen, mittels derer ein Bediener in komfortabler Weise die Zubereitung des Garguts steuern und überwachen kann.

In letzter Zeit hat sich im Rahmen der fortschreitenden Computerisierung ein Trend entwickelt, die Zubereitung von Speisen in Küchenmaschinen zu automatisieren. So ist es beispielsweise bekannt, dass der Bediener dem Display der Küchenmaschine Zubereitungsanweisungen entnehmen kann, die dann durch den Bediener zur entsprechenden Zubereitung der Speisen umgesetzt werden müssen, so durch Betätigen vorgenannter Schalter oder Tasten und/oder Befüllen des Rührgefäßes mit den benötigten Zutaten der Speisen, oder dass die Küchenmaschine ein Steuerprogramm zur selbsttätigen Abarbeitung von Zubereitungsschritten aufweist.

Die Druckschrift DE 10 2010 060 650 A1 offenbart beispielsweise eine Küchenmaschine mit einem selbsttätigen Abarbeitungsmodus für eine Abfolge von vorprogrammierten Zubereitungsschritten. Die Küchenmaschine und/oder ein Mobilgerät zur Zusammenwirkung mit der Küchenmaschine weisen eine Software auf, mit welcher bestimmte Rahmenbedingungen, wie beispielsweise die Wahl einer Rührzeit in Abhängigkeit von einer vorgegebenen Zutat, geändert oder ergänzt werden können. Das Dokument DE 10 2009 058 258 A1 offenbart ebenso eine Küchenmaschine, bei welcher in der Küchenmaschine selbst oder in einem mit der Küchenmaschine in Datenaustausch befindlichen Gerät eine Mehrzahl von Rezepten gespeichert sind. Die DE 10 2007 031 372 A1 offenbart, Rezepturen in Form von Folgen von mit der Küchenmaschine durchführbaren Bearbeitungsschritten über externe Einheiten wie Rechnereinheiten einzugeben und zu speichern.

Steuerprogramme für Küchenmaschinen sind in der Regel in einem nicht flüchtigen Speicher der Küchenmaschine abgelegt und somit bereits unmittelbar nach Erwerb der Küchenmaschine benutzbar. Dies ist unter anderem dadurch bedingt, dass die Erstellung der Steuerprogramme sehr aufwändig ist. Denn im Gegensatz zur Erstellung von sonstigen Computerprogrammen, die im schlimmsten Fall bei Fehlprogrammierung zu einem sogenannten "blue-screen", also Systemabsturz, führen können, führt ein fehlerhaftes Steuerprogramm einer Küchenmaschine im einfachsten Fall schlichtweg zur Ungenießbarkeit der zubereiteten Speisen. Im schlimmsten Fall kann ein fehlerhaftes Steuerprogramm dazu führen, dass das Rührwerk unkontrolliert in Bewegung gesetzt wird und Sicherheitsvorkehrungen außer Kraft gesetzt werden, so dass Verletzungen des Bedieners zu befürchten sind.

Vor diesem Hintergrund ist es Aufgabe der Erfindung, eine Anordnung anzugeben, mittels derer in besonders einfacher und zudem sicherer Weise ein Steuerprogramm zur automatisierten Zubereitung einer Küchenmaschine erstellt und durch die Küchenmaschine abgearbeitet werden kann.

Die Aufgabe wird durch die Merkmale des Anspruchs 1 gelöst. Vorteilhafte Ausgestaltungen und Weiterbildungen der Erfindung sind in den Unteransprüchen angegeben.

Demnach erfolgt die Lösung der Aufgabe durch eine Anordnung, mit einem Computersystem und einer Küchenmaschine, wobei das Computerprogramm eine Datenverarbeitungseinrichtung aufweist, und wobei die Küchenmaschine einen Mikroprozessor, ein aufheizbares Rührgefäß und ein in dem Rührgefäß befindliches Rührwerk aufweist, wobei der Mikroprozessor eingerichtet ist, anhand eines erstellten Steuerprogramms das Rührwerk und/oder die Heizung zu steuern, wobei auf der Datenverarbeitungseinrichtung ein computer-implementiertes Verfahren als Webapplikation ausgeführt wird, welches zum Erstellen eines als Datenstruktur ausgestalteten Steuerprogramms für die Küchenmaschine eingerichtet ist, wobei das Steuerprogramm eine automatisierte Zubereitung einer Speise durch eine Zubereitungsanweisung der Küchenmaschine ermöglicht, wobei die Datenverarbeitungseinrichtung zum Ausführen der nachfolgenden Schritte des Verfahrens ausgestaltet ist:
a) Erfassen wenigstens einer Zutat der Speise durch ein Eingabemittel,
b) Speichern der Zutat in einem ersten Datenarray durch die Datenverarbeitungseinrichtung, wobei jeder einzelnen Zutat ein einzelnes Datenfeld in dem ersten Datenarray zugeordnet ist,
c) Erfassen wenigstens einer Zeichenfolge, die einen Abarbeitungsschritt des Steuerprogramms darstellt, durch das Eingabemittel,
d) Speichern der Zeichenfolge in einem zweiten Datenarray durch die Datenverarbeitungseinrichtung, wobei jeder Abarbeitungsschritt in jeweils einem einzelnen Datenfeld des zweiten Datenarrays abgespeichert ist,
e) Prüfen durch Zeichenvergleich, ob die Zeichenfolge ein erstes Zeichen enthält, das einer Zutat entspricht und/ oder ob die Zeichenfolge ein zweites Zeichen enthält, das einer Zubereitungsanweisung entspricht durch die Datenverarbeitungseinrichtung, wobei die Zeichenfolge zunächst mittels eines Parsers untersucht wird, um mittels Zerlegung und/ oder Umwandlung der Zeichenfolge ein für die Weiterverarbeitung brauchbares Format zu erhalten, damit danach die Prüfung durch Zeichenvergleich durchführbar ist,
f) Verlinken des in dem zweiten Datenarray gespeicherten ersten Zeichens mit der in dem ersten Datenarray gespeicherten Zutat und Verlinken des in dem zweiten Datenarray gespeicherten zweiten Zeichens mit der Zubereitungsanweisung durch die Datenverarbeitungseinrichtung,
h) Prüfen, ob jede in Schritt a) erfasste Zutat durch Schritt f) verlinkt worden ist durch die Datenverarbeitungseinrichtung,
k) Erstellen des Steuerprogramms durch Compilieren der verlinkten Zeichenfolge durch die Datenverarbeitungseinrichtung,
l) Übertragen des Steuerprogramms auf die Küchenmaschine durch die Datenverarbeitungseinrichtung,
m) Ausführen des Steuerprogramms durch den Mikroprozessor der Küchenmaschine, und
n) Steuern des Rührwerks und/ oder der Heizung der Küchenmaschine entsprechend des Steuerprogramms durch den Mikroprozessor zum automatisierten Zubereiten der Speise.

Die Erfindung geht damit einen ganz neuen Weg, indem eine Anordnung zur automatisierten Erstellung und Abarbeitung eines Steuerprogramms für die automatisierte Speisenzubereitung mittels einer Küchenmaschine vorgeschlagen wird. Das Verfahren ist als Web-Applikation ausgestaltet und ablauffähig in einem Web-Browser des Bedieners, ermöglicht zunächst die Erfassung der Zutaten der gewünschten zuzubereitenden Speise durch das Eingabemittel, so beispielsweise eine Tastatur, durch den Schritt a), wobei die Zutat exemplarisch "Zwiebeln" lauten kann.

In der Folge wird die mittels des Eingabemittels eingegebene Zutat in einem ersten Datenarray gespeichert. Das Datenarray ist bevorzugt Teil einer Datenbank, so beispielsweise einer in SQL oder Oracle programmierten Datenbank. Sofern nun, wie auch in der Regel üblich, die zuzubereitende Speise eine Mehrzahl von Zutaten aufweist, können bevorzugt die Schritte a) und b) wiederholt durchgeführt werden, bis dass alle Zutaten erfasst und in dem ersten Datenarray abgespeichert sind. Dabei ist jeder einzelnen Zutat ein einzelnes Datenfeld in dem ersten Datenarray zugeordnet.

Ein wesentlicher Punkt der Erfindung ist, dass gemäß Schritt c) eine Zeichenfolge durch das Computer-implementierte Verfahren erfasst wird, so beispielsweise ebenso mittels vorgenannter Tastatur. Die Zeichenfolge stellt einen Abarbeitungsschritt des Steuerprogramms dar, wobei die Zeichenfolge bevorzugt als sogenannter Fließtext eingegeben und mittels Schritt d) in einem zweiten Datenarray gespeichert wird. Da die Zubereitung einer Speise nicht selten eine Mehrzahl von Abarbeitungsschritten aufweist, in der Summe auch Rezept genannt, werden die Schritte c) und d) bevorzugt wiederholt ausgeführt, bis dass alle Abarbeitungsschritte durch das Eingabemittel erfasst und bevorzugt in jeweils einem einzelnen Datenfeld des zweiten Datenarrays abgespeichert sind. Das zweite Datenarray ist dazu wie das erste Datenarray bevorzugt in der Datenbank vorgesehen. Dadurch, dass die Abarbeitungsschritte als Fließtext eingegeben werden können, gestaltet sich die Bedienung des Computer-implementierten Verfahrens für den Bediener besonders einfach. Eine durch einen Bediener getätigte Eingabe kann beispielsweise "Lege Zwiebeln in das Rührgefäß und lasse das Rührwerk 5 Sekunden auf Geschwindigkeit 7 laufen" lauten.

Danach wird im Schritt e) geprüft, ob die Zeichenfolge ein erstes Zeichen enthält, das einer Zutat entspricht und ob die Zeichenfolge ein zweites Zeichen enthält, das einer Zubereitungsanweisung der Küchenmaschine entspricht. Dem vorgenannten Beispiel folgend, enthält die Zeichenfolge zunächst die Zutat "Zwiebeln" sowie die Zubereitungsanweisungen "5 Sekunden" sowie "Geschwindigkeit 7". Das Computer-implementierte Verfahren untersucht die Zeichenfolge während des Schrittes e) zunächst mittels eines Parsers, um mittels Zerlegung und/oder Umwandlung der Zeichenfolge ein für die Weiterverarbeitung brauchbares Format zu erhalten, damit danach die in Schritt e) beanspruchte Prüfung durch Zeichenvergleich durchführbar ist.

Nachdem nun die Zeichenfolge bzw. vorzugsweise alle erfassten Zeichenfolgen gemäß dem Schritt e) geprüft worden sind, erfolgt in Schritt f) das Verlinken des in dem zweiten Datenarray gespeicherten ersten Zeichens, welches zuvor mittels Schritt e) erkannt worden ist, mit der in dem ersten Datenarray gespeicherten Zutat und/oder das Verlinken des in dem zweiten Datenarray gespeicherten zweiten Zeichens, welches ebenso mittels des vorherigen Schrittes e) erkannt worden ist, mit der Zubereitungsanweisung.

Die Verlinkung erfolgt dabei vorzugsweise innerhalb der Datenbank, wobei jedoch ebenso eine Verlinkung mittels Hyperlink oder einer vergleichbaren Möglichkeit durchgeführt werden kann. Im Ergebnis wird die zuvor als Fließtext eingebbare Zeichenfolge mit den bereits vorab eingegebenen Zutaten der Speise oder der Zubereitungsanweisungen der Küchenmaschine durch die Verlinkung verknüpft, so dass aus der so erhaltenen Verknüpfung von Zutaten mit Abarbeitungsschritten bzw. Zubereitungsanweisungen das Steuerprogramm zur automatisierten Zubereitung der Speise in einem Computerverarbeitbaren Format ableitbar ist.

In Schritt h) findet schließlich eine Konsistenzprüfung statt, ob jede in Schritt a) erfasste Zutat durch den Schritt f) verlinkt worden ist, so also ob für jede Zutat in dem Steuerprogramm eine Verknüpfung durch Verlinkung vorliegt. Dadurch kann sichergestellt werden, dass tatsächlich alle Zutaten im zu erstellenden Steuerprogramm berücksichtigt worden sind. Darüber hinaus kann es möglich sein, dass neben Zutaten auch weitere Anweisungen, beispielsweise Servieranweisungen, erfasst werden, die dann ebenso verlinkt werden können, so dass auch für die Servieranweisungen vorgenannte Konsistenzprüfung durchgeführt werden kann.

Im Ergebnis ermöglicht das in Rede stehende Verfahren eine automatisierte und besonders einfache Erstellung eines Steuerprogramms einer Küchenmaschine, welches in einem weiteren Schritt nach Erstellung und Übertragung auf die Küchenmaschine die automatisierte Zubereitung der Speisen auf der Küchenmaschine ermöglicht.

Nach einer bevorzugten Ausführungsform umfasst das Verfahren ferner den Schritt: g) Wiederholen des Schrittes f), bis dass alle erste Zeichen der Zeichenfolge, die einer Zutat entsprechen, mit einer in dem ersten Datenarray gespeicherten Zutat verlinkt sind und/oder bis dass alle zweite Zeichen der Zeichenfolge, die einer Zubereitungsanweisung entsprechen, mit einer Zubereitungsanweisung verlinkt sind. Bevorzugt erfolgt die Wiederholung des Schrittes f) so oft, wie durch den Schritt a) unterschiedliche Zutaten erfasst worden sind, damit sichergestellt ist, damit jede Zutat in der Zeichenfolge erkannt worden ist.

Sollte bei der Wiederholung des Schrittes f) festgestellt werden, dass nicht alle erfassten Zutaten verlinkt worden sind, so insbesondere weil nicht alle Zutaten durch den Schritt e) in der erfassten Zeichenfolge bzw. in den erfassten Zeichenfolgen erkannt worden sind, so kann das Verfahren eine Fehlermeldung ausgeben und den Bediener beispielsweise zur Erfassung weiterer Zeichenfolgen auffordern. Dabei können dem Bediener auch die Zutaten auf einem Display dargestellt werden, die noch nicht in den Zeichenfolgen mittels des Schrittes e) erkannt worden sind.

Nach einer weiteren bevorzugten Ausgestaltung ist vorgesehen, dass das Verfahren ferner den Schritt i) aufweist, so ein Prüfen, ob jedes in Schritt e) gefundene zweite Zeichen mit einer Zubereitungsanweisung verlinkt ist. Auch hier kann vorgesehen sein, dass das Verfahren eine Fehlermeldung ausgibt, so dass der Bediener die in Schritt c) erfasste Zeichenfolge editieren und/oder weitere Zeichenfolgen in das Eingabemittel eingeben kann. In jedem Fall wird durch diesen Schritt eine weitere Konsistenzprüfung ermöglicht, ob denn tatsächlich auch alle Zubereitungsanweisungen richtig erkannt und verlinkt worden sind.

Nach einer ganz besonders bevorzugten Ausgestaltung ist vorgesehen, dass gemäß dem Schritt c') ein Auswahlmenü zum Auswählen der in Schritt a) erfassten Zutat und/oder der Zubereitungsanweisung der Küchenmaschine bereitgestellt wird. Besonders bevorzugt erfolgt die Bereitstellung gemäß Schritt c') während des Schrittes c). Dem Bediener kann im Rahmen eines Auswahlmenüs, so beispielsweise eines Scroll-Down-Menüs, eine besonders einfache Option angegeben werden, mittels derer er die vorab eingegebenen Zutaten, so gegebenenfalls auch anteilig, in simpler Weise auswählen kann, so beispielsweise durch Klicken auf die entsprechende Zutat.

Ebenso kann vorgesehen sein, dass der Bediener durch das Auswahlmenu die vorzugsweise zuvor definierten Zubereitungsanweisungen der Küchenmaschine auswählen kann, so beispielsweise hinsichtlich Geschwindigkeit, Temperatur oder Zeitdauer, des Rührwerkes, der Heizung etc., die in der Regel abhängig von der designierten Küchenmaschine sind, auf dem das Steuerprogramm später ablaufen soll. Ferner kann das Computer-implementierte Verfahren Variablen aufweisen, die die grundsätzlich bei einem bestimmten Typ der Küchenmaschine verfügbaren und damit auswählbaren Zubereitungsanweisungen definieren. In diesem Zusammenhang ist ganz besonders bevorzugt, dass die Zubereitungsanweisung aus den Parametern Zeit, Geschwindigkeit, Temperatur, Wiegen und/oder Dampfgaren auswählbar ist. Sofern die Küchenmaschine mit unterschiedlichen Rührwerken, Rührgefäßen und/oder Erweiterungen vorgenannter Komponenten konfektionierbar ist, können sich dadurch weitere Parameter ergeben.

Nach einer weiteren bevorzugten Ausführungsform ist vorgesehen, dass in Schritt a) zu jeder Zutat die Menge der Zutat und die Maßeinheit der Menge erfasst werden. Die Menge kann beispielsweise einen numerischen Wert darstellen, während die Maßeinheit in "Gramm", "Teelöffel", "ml" oder ähnlichem ausgedrückt sein kann. Nach einer weiteren bevorzugten Ausführungsform weist das Verfahren einen weiteren Prüfungsschritt auf, mittels dem geprüft wird, ob die erfasste Menge mit der Küchenmaschine verarbeitbar ist, so beispielsweise ob die erfasste Menge größer ist als das Rührgefäß der Küchenmaschine. Für diesen Fall kann vorgesehen sein, dass das Verfahren automatisch eine Fehlermeldung ausgibt und der Bediener aufgefordert wird, die Menge zu korrigieren. Ebenso kann vorgesehen sein, dass die Gesamtmenge aller eingegebenen Zutaten hinsichtlich der maximalen durch die Küchenmaschine verarbeitbaren Menge geprüft wird. Zur einfachen Handhabung sind die Menge und/oder die Maßeinheit aus einem Menü auswählbar, wobei andererseits auch vorgesehen sein kann, dass die Menge und/oder die Maßeinheit mittels einer Tastatur als Eingabemittel erfasst werden können.

Ebenso kann nach einer weiteren bevorzugten Ausgestaltung vorgesehen sein, dass das Verfahren den Schritt f') Ausgeben der Zeichenfolge mit Darstellung der verlinkten Zeichen und/oder den Schritt j) Bereitstellen eines Editiermittels zum Editieren der ausgegebenen Zeichenfolge aufweist. Bevorzugt erfolgt dabei die Darstellung der verlinkten Zeichen durch optische Hervorhebung der Verlinkung, so beispielsweise durch Unterstreichung und/oder Fettschrift. Durch das Editiermittel, welches durch eine aktivierbare Menüfunktion ausgestaltet sein kann, erhält der Bediener die Möglichkeit, möglicherweise falsch eingegebene Zeichenfolgen zu ändern, zu ergänzen und/oder zu löschen. In jedem Fall wird durch vorgenannte Ausgestaltungen die Bedienung des Verfahrens sehr benutzerfreundlich ausgestaltet.

Nachdem nun der Bediener alle Zutaten und alle Zeichenfolgen mittels der Eingabemittel eingegeben hat, und das Verfahren alle Verlinkungen und Prüfungen erfolgreich durchgeführt hat, erfolgt danach gemäß den Schritten k) die Erstellung des Steuerprogramms durch Compilieren der verlinkten Zeichenfolge, und gemäß Schritt 1) das Übertragen des Steuerprogramms auf die Küchenmaschine. Wie bereits vorab ausgeführt, sind bevorzugt das erste Datenarray und das zweite Datenarray in einer Datenbank abgelegt, wobei durch die Zeichenfolge und entsprechende Verlinkung gemäß Schritt f) die Abarbeitungsschritte des Steuerprogramms erfasst worden sind. Nachdem nun vorgenannte Schritte erfolgreich durchlaufen worden sind, liegt das Steuerprogramm, so als Rezept zur automatischen Abarbeitung, bevorzugt in der Datenbank vor.

Um nun das Steuerprogramm auf der Küchenmaschine auszuführen, bedarf es gemäß dem Schritt k) zunächst eines Kompiliervorgangs. Die Küchenmaschine weist bevorzugt einen Mikroprozessor, auf, wobei das Compilieren bevorzugt derart erfolgt, dass der Kompilierer einen durch den Mikroprozessor der Küchenmaschine ausführbaren Code erstellt. Die Übertragung des Steuerprogramms auf die Küchenmaschine erfolgt vorzugsweise drahtgebunden oder kabellos über ein Netzwerk, oder beispielsweise mittels eines USB-Sticks oder ähnlichem Mittel. Nach der Übertragung des Steuerprogramms auf die Küchenmaschine kann der Mikroprozessor der Küchenmaschine das kompilierte Steuerprogramm zur automatisierten Zubereitung der Speise ausführen, so nach Erhalt einer Startanweisung durch den Bediener.

Nachfolgend wird die Erfindung unter Bezugnahme auf die anliegende Zeichnung anhand einer bevorzugten Ausführungsform näher erläutert.

Es zeigen
- Fig. 1: eine Küchenmaschine mit einer Datenverarbeitungseinrichtung zur Ausführung des Verfahrens gemäß der bevorzugten Ausführungsform, und
- Fig. 2: ein Ablaufdiagramm zur Durchführung des Verfahrens gemäß der bevorzugten Ausführungsform.

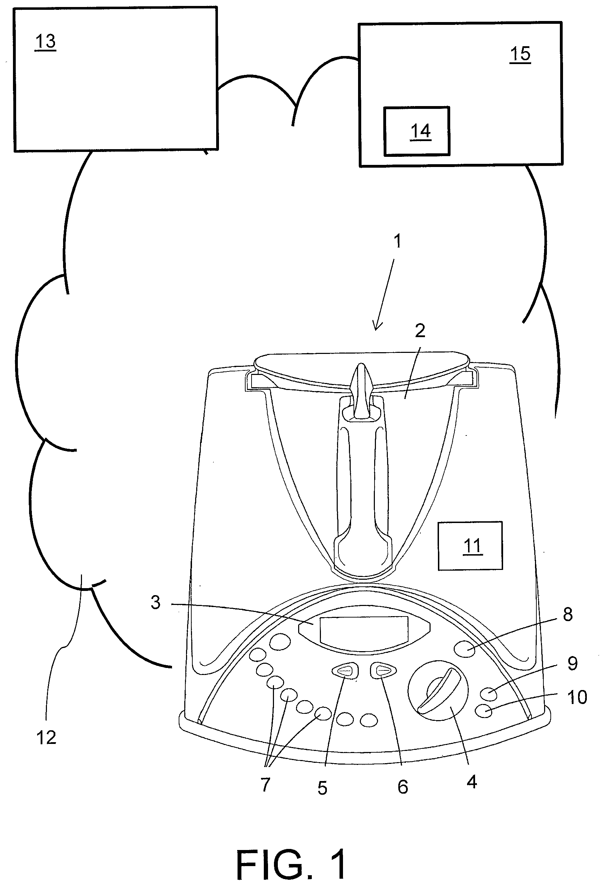

Fig. 1 zeigt eine Küchenmaschine 1, wie sie auch als "Vorwerk-Thermomix" bekannt ist.

Die Küchenmaschine 1 weist ein Rührgefäß 2 auf, welches ein am Boden des Rührgefäßes 2 angeordnetes, nicht sichtbares Rührwerk aufweist. Das Rührgefäß 2 ist aufheizbar, so mittels einer nicht dargestellten bodenseitig im Rührgefäß 2 vorgesehenen elektrischen Widerstandsheizung. An der Küchenmaschine 1 ist ein Display 3 vorgesehen, welches unter anderem die Drehgeschwindigkeit des Rührwerkes und die Temperatur der Widerstandsheizung anzeigt.

Zudem sind verschiedene Bedienelemente zur manuellen Zubereitung von Speisen vorgesehen, so ein Drehwahlschalter 4 zur Einstellung einer Rührwerksgeschwindigkeit, Betätigungstasten 5, 6 für eine Zeiteinstellung, Tasten 7 zur Temperaturvorwahl der Widerstandsheizung sowie weitere Schalter 8 bis 10, mittels derer weitere Funktionalität der Küchenmaschine 1 einstellbar ist, so wie etwa eine Turbofunktion, eine Wiegefunktion oder dergleichen.

Um nun eine Speise manuell zuzubereiten, sind die Zutaten der Speise in das Rührgefäß 2 zu füllen, worauf dann mittels der Betätigungstasten 5, 6, 7 und/oder Schalter 8 bis 10 die entsprechende Funktion der Küchenmaschine 1 zur Zubereitung der Speise einzustellen ist. Neben vorgenannter manueller Zubereitung der Speise verfügt die Küchenmaschine 1 über einen Mikroprozessor 11, der die automatisierte Zubereitung der Speise anhand eines Steuerprogramms steuert. Das Steuerprogramm ist mittels eines Netzwerkes 12, vorliegend als Drahtlosnetzwerk ausgeführt, von einer als Computer ausgestalteten Datenverarbeitungseinrichtung 13 auf die Küchenmaschine 1 übertragbar.

Auf der Datenverarbeitungseinrichtung 13 wird ein Computer-implementiertes Verfahren als Web-Applikation ausgeführt, welches zum Erstellen des Steuerprogramms der Küchenmaschine 1 derart eingerichtet ist, dass anhand des fertig gestellten und auf die Küchenmaschine 1 übertragenen Steuerprogramms die automatisierte Zubereitung der Speise durch eine Zubereitungsanweisung der Küchenmaschine ermöglicht wird.

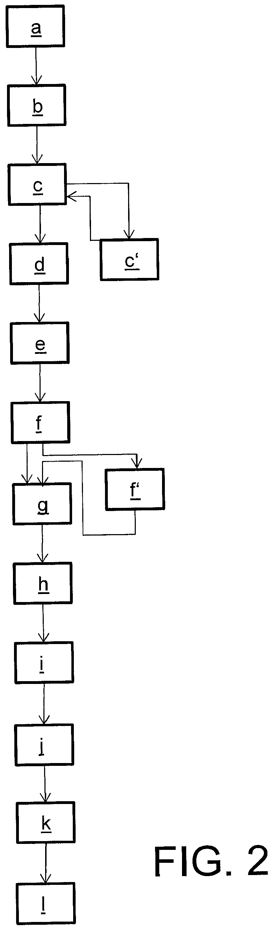

Das Verfahren, dargestellt anhand eines Ablaufdiagramms in Fig. 2, weist dazu verschiedene Schritte auf, die nacheinander ausgeführt werden und im Folgenden detailliert sind:
In Schritt a) folgt zunächst das Erfassen wenigstens einer Zutat der Speise durch ein Eingabemittel 14, so durch eine Tastatur eines Computers 15 eines Bedieners, der auf seinem Computer 15 die auf der Datenverarbeitungseinrichtung 13 ausgeführte Web-Applikation aufgerufen hat. Neben der Zutat, die vorliegend als "Zwiebeln" eingegeben wird, erfolgt noch die Erfassung der Menge der Zutat, so "200", und die Maßeinheit der Menge, so "g" für Gramm. Im Ergebnis ist damit "200 g Zwiebeln" erfasst worden.

Nach Eingabe der Zutat erfolgt in Schritt b) das Speichern der Zutat in einem ersten Datenarray auf der Datenverarbeitungseinrichtung 13, so in einer auf der Datenverarbeitungseinrichtung 13 ablaufenden Datenbank. Die Schritte a) und b) wiederholt der Bediener, bis dass alle Zutaten der zuzubereitenden Speise eingegeben sind.

In Schritt c) erfolgt dann das Erfassen wenigstens einer Zeichenfolge, die einen Abarbeitungsschritt des Steuerprogramms darstellt. Das bedeutet, dass der Bediener mittels des als Tastatur ausgestalteten Eingabemittels 14 einen Freitext in die Web-Applikation eingibt, der einen Abarbeitungsschritt des Steuerprogramms darstellt. Dieser Freitext ist vorliegend "Lege Zwiebeln in das Rührgefäß und lasse das Rührwerk 5 Sekunden auf Geschwindigkeit 7 laufen". Die Zeichenfolge wird gemäß Schritt d) in einem zweiten Datenarray der Datenbank gespeichert.

Um die Erfassung der Zeichenfolge zu vereinfachen, weist die Web-Applikation ein Auswahlmenü auf, mittels derer die in Schritt a) erfassten Zutaten und eine Zubereitungsanweisung der Küchenmaschine durch den Bediener ausgewählt werden können, so beispielsweise mittels einer Computermaus in einem Pop-down- oder Auswahlmenü.

Dabei ist unter dem Begriff Zubereitungsanweisung im Rahmen der Erfindung ein Parameter der Küchenmaschine 1 gemeint, mittels derer die Zubereitung der Speise steuerbar ist, so Zeit, also wie lange das Rührwerk oder die Widerstandsheizung auf die Zutat einwirken soll, Geschwindigkeit, so des Rührwerkes, Temperatur, so der Widerstandsheizung, Wiegen, so Durchführung einer Wiegefunktion der Küchenmaschine 1, und/oder Dampfgaren mittels des Rührgefäßes 2. Wie eingangs diskutiert, sind derartige Zubereitungsanweisungen ebenso manuell durch den Drehwahlschalter 4, die Betätigungstasten 5, 6, 7 sowie die Schalter 8 bis 10 einstellbar.

In Schritt e) erfolgt durch das Computer-implementierte Verfahren eine Prüfung, ob die Zeichenfolge ein erstes Zeichen enthält, das einer Zutat entspricht und/oder ob die Zeichenfolge ein zweites Zeichen enthält, das einer Zubereitungsanweisung entspricht. Der vorab eingebende Fließtext wird dafür zunächst durch einen Parser zerlegt und in ein für die Datenverarbeitungseinrichtung 13 verarbeitbares Format konvertiert, so dass dann die zerlegten Bestandteile der Zeichenfolge mit den vorab durch Schritt a) eingegebenen Zutaten sowie den Zubereitungsanweisungen verglichen werden können. Vorliegend ist das Ergebnis der Prüfung, dass einerseits die Zutat "Zwiebeln" aufgefunden wird und andererseits die Zubereitungsanweisung "5 Sekunden" sowie "Geschwindigkeit 7" festgestellt wird.

Die so durch den Schritt e) festgestellten ersten Zeichen und zweiten Zeichen werden dann in Schritt f) mit den Zutaten bzw. Zubereitungsanweisungen verlinkt. Das bedeutet, dass die in dem zweiten Datenarray gespeicherten und im Vorschritt festgestellten Zeichen mit der in dem ersten Datenarray gespeicherten Zutat verlinkt werden und die im zweiten Datenarray gespeicherten im Vorschritt festgestellten zweiten Zeichen mit der jeweiligen Zubereitungsanweisung verlinkt werden. Derartiges Verlinken erfolgt durch Hinzufügung eines Hyperlinks zu der Zeichenfolge, welche gemäß Schritt f') auf einem Display des Computers 15 mit Darstellung der verlinkten Zeichen ausgebbar ist.

Gemäß Schritt g) wird nunmehr der Schritt f) wiederholt, bis dass alle ersten Zeichen der Zeichenfolge, die einer Zutat entsprechen, mit einer in dem ersten Datenarray gespeicherten Zutat verlinkt sind und bis dass alle zweite Zeichen der Zeichenfolge, die einer Zubereitungsanweisung entsprechen, mit einer Zubereitungsanweisung verlinkt sind.

Danach wird in Schritt h) überprüft, ob jede in Schritt a) erfasste Zutat durch den Schritt f) verlinkt worden ist. Zudem erfolgt in Schritt i) eine Prüfung, ob jedes in Schritt e) gefundene zweite Zeichen mit einer Zubereitungsanweisung verlinkt ist. Anders formuliert erfolgt durch vorgenannte Schritte eine Konsistenzprüfung, ob denn jeder vorab eingegebenen Zutat und auch jede Zubereitungsanweisung verlinkt worden sind.

Danach erhält der Bediener mittels des Schrittes j) nochmals die Möglichkeit, die eingegebenen bzw. durch Schritt f) ausgegebenen Zeichenfolgen zu editieren, so beispielsweise wenn sich eine Änderung einer Zubereitungsanweisung bzw. eines Rezeptschrittes ergeben hat.

Schließlich erfolgt durch die Schritte k) die Erstellung des Steuerprogramms durch Kompilieren der verlinkten Zeichenfolgen in ein durch den Mikroprozessor 11 der Küchenmaschine 1 ausführbares Steuerprogramm, welches dann mittels Schritt 1) von der Datenverarbeitungseinrichtung 13 mittels des Netzwerkes 12 auf die Küchenmaschine 1 übertragen wird.

## Patentansprüche

1. Anordnung, mit einem Computersystem und einer Küchenmaschine (1), wobei das Computersystem eine Datenverarbeitungseinrichtung (13) aufweist, und wobei
die Küchenmaschine (1) einen Mikroprozessor (11), ein aufheizbares Rührgefäß (2) und ein in dem Rührgefäß (2) befindliches Rührwerk aufweist, wobei der Mikroprozessor (11) eingerichtet ist, anhand eines erstellten Steuerprogramms das Rührwerk und/ oder die Heizung zu steuern, **dadurch gekennzeichnet, dass** auf der Datenverarbeitungseinrichtung (13) ein computer-implementiertes Verfahren als Webapplikation ausgeführt wird, welches zum Erstellen eines als Datenstruktur ausgestalteten Steuerprogramms für die Küchenmaschine (1) eingerichtet ist, wobei das Steuerprogramm eine automatisierte Zubereitung einer Speise durch eine Zubereitungsanweisung der Küchenmaschine (1) ermöglicht,
wobei die Datenverarbeitungseinrichtung (13) zum Ausführen der nachfolgenden Schritte des Verfahrens ausgestaltet ist:
a) Eingabe wenigstens einer Zutat der Speise durch ein Eingabemittel (14),
b) Speichern der Zutat in einem ersten Datenarray durch die Datenverarbeitungseinrichtung (13), wobei jeder einzelnen Zutat ein einzelnes Datenfeld in dem ersten Datenarray zugeordnet ist,
c) Eingabe wenigstens einer Zeichenfolge, die einen Abarbeitungsschritt des Steuerprogramms darstellt, durch das Eingabemittel (14),
d) Speichern der Zeichenfolge in einem zweiten Datenarray durch die Datenverarbeitungseinrichtung (13), wobei jeder Abarbeitungsschritt in jeweils einem einzelnen Datenfeld des zweiten Datenarrays abgespeichert ist.
e) Prüfen durch Zeichenvergleich, ob die Zeichenfolge ein erstes Zeichen enthält, das einer Zutat entspricht und ob die Zeichenfolge ein zweites Zeichen enthält, das einer Zubereitungsanweisung entspricht durch die Datenverarbeitungseinrichtung (13), wobei die Zeichenfolge zunächst mittels eines Parsers untersucht wird, um mittels Zerlegung und/ oder Umwandlung der Zeichenfolge ein für die Weiterverarbeitung brauchbares Format zu erhalten, damit danach die Prüfung durch einen Zeichenvergleich durchführbar ist,
f) Verlinken des in dem zweiten Datenarray gespeicherten ersten Zeichens mit der in dem ersten Datenarray gespeicherten Zutat und Verlinken des in dem zweiten Datenarray gespeicherten zweiten Zeichens mit der Zubereitungsanweisung durch die Datenverarbeitungseinrichtung (13),
h) Prüfen, ob jede in Schritt a) erfasste Zutat durch Schritt f) verlinkt worden ist durch die Datenverarbeitungseinrichtung (13),
k) Erstellen des Steuerprogramms durch Compilieren der verlinkten Zeichenfolge durch die Datenverarbeitungseinrichtung (13),
l) Übertragen des Steuerprogramms auf die Küchenmaschine (1) durch die Datenverarbeitungseinrichtung (13),
m) Ausführen des Steuerprogramms durch den Mikroprozessor (11) der Küchenmaschine (1), und
n) Steuern des Rührwerks und/ oder der Heizung der Küchenmaschine (1) entsprechend des Steuerprogramms durch den Mikroprozessor (11) zum automatisierten Zubereiten der Speise.

2. Anordnung nach dem vorhergehenden Anspruch, wobei die Datenverarbeitungseinrichtung (13) zum Ausführen des nachfolgenden Schrittes ausgestaltet ist:
g) Wiederholen des Schrittes f), bis dass alle erste Zeichen der Zeichenfolge, die einer Zutat entsprechen, mit einer in dem ersten Datenarray gespeicherten Zutat verlinkt sind und/ oder bis dass alle zweite Zeichen der Zeichenfolge, die einer Zubereitungsanweisung entsprechen, mit einer Zubereitungsanweisung verlinkt sind.

3. Anordnung nach einem der vorhergehenden Ansprüche, wobei die Datenverarbeitungseinrichtung (13) zum Ausführen des nachfolgenden Schrittes ausgestaltet ist:
i) Prüfen, ob jedes in Schritt e) gefundene zweite Zeichen mit einer Zubereitungsanweisung verlinkt ist.

4. Anordnung nach einem der vorhergehenden Ansprüche, wobei die Datenverarbeitungseinrichtung (13) zum Ausführen des nachfolgenden Schrittes ausgestaltet ist:
c') Bereitstellen eines Auswahlmenues zum Auswählen der in Schritt a) erfassten Zutat und/oder der Zubereitungsanweisung der Küchenmaschine (1).

5. Anordnung nach dem vorhergehenden Anspruch, wobei die Zubereitungsanweisung aus den Parametern Zeit, Geschwindigkeit, Temperatur, Wiegen und/oder Dampfgaren auswählbar ist.

6. Anordnung nach einem der vorhergehenden Ansprüche, wobei in Schritt a) zu jeder Zutat die Menge der Zutat und die Maßeinheit der Menge erfasst werden.

7. Anordnung nach einem der vorhergehenden Ansprüche, wobei die Datenverarbeitungseinrichtung (13) zum Ausführen des nachfolgenden Schrittes ausgestaltet ist:
f') Ausgeben der Zeichenfolge mit Darstellung der verlinkten Zeichen.

8. Anordnung nach einem der vorhergehenden Ansprüche, wobei die Datenverarbeitungseinrichtung (13) zum Ausführen des nachfolgenden Schrittes ausgestaltet ist:
j) Bereitstellen eines Editiermittels zum Editieren der ausgegebenen Zeichenfolge.

9. Anordnung nach einem der vorhergehenden Ansprüche, wobei die Küchenmaschine (1) eine Waage aufweist, und der Mikroprozessor (11) eingerichtet ist, anhand des erstellten Steuerprogramms die Waage zu steuern.

## Claims

1. Arrangement comprising a computer system and a kitchen appliance (1), the computer system comprising a data processing device (13), and
the kitchen appliance (1) comprising a microprocessor (11), a heatable stirring vessel (2) and a stirring mechanism located in the stirring vessel (2), the microprocessor (11) being set up to control the stirring mechanism and/or the heater using a generated control program, **characterised in that** a computer-implemented method is executed as a web application on the data processing device (13), which method is set up to generate a control program for the kitchen appliance (1) that is designed as a data structure, the control program allowing automated preparation of a meal based on a preparation instruction of the kitchen appliance (1),
the data processing device (13) being configured to execute the following steps of the method:
a) inputting at least one ingredient of the meal by means of an input means (14),
b) storing the ingredient in a first data array by means of the data processing device (13), an individual data field in the first data array being assigned to each individual ingredient,
c) inputting at least one character string that represents a processing step of the control program, by means of the input means (14),
d) storing the character string in a second data array by means of the data processing device (13), each processing step being saved in each case in one individual data field of the second data array,
e) checking by means of character comparison whether the character string contains a first character, which corresponds to an ingredient, and whether the character string contains a second character, which corresponds to a preparation instruction, by means of the data processing device (13), the character string first being analysed using a parser in order to obtain, by means of decomposing and/or converting the character string, a format that can be used for further processing, such that checking can subsequently be carried out by means of a character comparison,
f) linking the first character stored in the second data array to the ingredient stored in the first data array and linking the second character stored in the second data array to the preparation instruction, by means of the data processing device (13),
h) checking whether every ingredient entered in step a) has been linked by step f), by means of the data processing device (13),
k) generating the control program by compiling the linked character string, by means of the data processing device (13),
l) transferring the control program to the kitchen appliance (1) by means of the data processing device (13),
m) executing the control program by means of the microprocessor (11) of the kitchen appliance (1), and
n) controlling the stirring mechanism and/or the heater of the kitchen appliance (1) in accordance with the control program, by means of the microprocessor (11), for the automated preparation of the meal.

2. Arrangement according to the preceding claim, wherein the data processing device (13) is configured to execute the following step of:
g) repeating step f) until all the first characters of the character string, which correspond to an ingredient, are linked to an ingredient stored in the first data array, and/or until all second characters of the character string, which correspond to a preparation instruction, are linked to a preparation instruction.

3. Arrangement according to either of the preceding claims, wherein the data processing device (13) is configured to execute the following step of:
i) checking whether every second character found in step e) is linked to a preparation instruction.

4. Arrangement according to any of the preceding claims, wherein the data processing device (13) is configured to execute the following step of:
c') providing a selection menu for selecting the ingredient entered in step a) and/or for selecting the preparation instruction of the kitchen appliance (1).

5. Arrangement according to the preceding claim, wherein the preparation instruction can be selected from the parameters of time, speed, temperature, weighing and/or steam cooking.

6. Arrangement according to any of the preceding claims, wherein the quantity of the ingredient and the unit of measure of the quantity are entered, for each ingredient, in step a).

7. Arrangement according to any of the preceding claims, wherein the data processing device (13) is configured to execute the following step of:
f') outputting the character string with a depiction of the linked characters.

8. Arrangement according to any of the preceding claims, wherein the data processing device (13) is configured to execute the following step of:
j) providing an editing means for editing the output character string.

9. Arrangement according to any of the preceding claims, wherein the kitchen appliance (1) comprises a scale, and the microprocessor (11) is set up to control the scale using the generated control program.

## Revendications

1. Dispositif comprenant un système informatique et un robot de cuisine (1), dans lequel le système informatique comprend un dispositif de traitement de données (13), et dans lequel le robot de cuisine (1) comprend un microprocesseur (11), un récipient de mélange (2) qui peut être chauffé, et un mécanisme d'agitation situé dans le récipient de mélange, dans lequel le microprocesseur (11) est configuré pour commander le mécanisme d'agitation et / ou le dispositif de chauffe à l'aide d'un programme de commande établi, **caractérisé en ce qu'**un procédé implémenté par ordinateur est exécuté sous la forme d'une application Web sur le dispositif de traitement de données (13), ledit procédé étant conçu pour établir un programme de commande en tant que structure de données pour le robot de cuisine (1), dans lequel le programme de commande permet une préparation automatisée d'un plat par une instruction de préparation du robot de cuisine (1),
dans lequel le dispositif de traitement de données (13) est configuré pour exécuter les étapes suivantes du procédé :
a) entrer au moins un ingrédient du plat par l'intermédiaire d'un dispositif d'entrée (14) ;
b) mémoriser l'ingrédient dans un premier tableau de données par le dispositif de traitement de données (13), chaque ingrédient individuel étant associé à un champ de données individuel dans le premier tableau de donnés,
c) entrer au moins une séquence de caractères qui représente une séquence de traitement du programme de commande par l'intermédiaire du dispositif d'entrée (14),
d) mémoriser la séquence de caractères dans un deuxième tableau de données par le dispositif de traitement de données (13), chaque séquence de traitement étant mémorisée dans un champ de données individuel du deuxième tableau de donnés,
e) vérifier, par comparaison de caractères, si la séquence de caractères contient un premier caractère correspondant à un ingrédient et si la séquence de caractères contient un deuxième caractère correspondant à une instruction de préparation par le dispositif de traitement de données (13), la séquence de caractères étant d'abord analysée au moyen d'un analyseur afin d'obtenir, au moyen de décomposition et / ou transformation de la séquence de caractères, un format utile pour le traitement ultérieur avec lequel la vérification est ensuite réalisable par comparaison de caractère,
f) relier le premier caractère mémorisé dans le deuxième tableau de données à l'ingrédient mémorisé dans le premier tableau de données, et relier le deuxième caractère mémorisé dans le deuxième tableau de données à l'instruction de préparation par le dispositif de traitement de données (13),
h) vérifier si chaque ingrédient entré à l'étape a) a été lié par l'étape f) par le dispositif de traitement de données (13),
k) générer le programme de commande en compilant la séquence de caractères liée par le dispositif de traitement de données (13),
l) transférer le programme de commande sur l'robot de cuisine (1) par le dispositif de traitement de données (13),
m) exécuter le programme de commande par le microprocesseur (11) du robot de cuisine (1), et
n) commander le mécanisme d'agitation et / ou le dispositif de chauffe du robot de cuisine (1) conformément au programme de commande par le microprocesseur (11) pour la préparation automatisée du plat.

2. Dispositif selon la revendication 1, dans lequel le dispositif de traitement de données (13) est configuré pour exécuter l'étape suivante :
g) répéter l'étape f) jusqu'à ce que tous les premiers caractères de la séquence de caractères qui correspondent à un ingrédient, soient liés à un ingrédient mémorisé dans le premier tableau de données, et / ou jusqu'à ce que tous les deuxièmes caractères de la séquence de caractères qui correspondent à une instruction de préparation, soient liés à une instruction de préparation.

3. Dispositif selon l'une quelconque des revendications précédentes, dans lequel le dispositif de traitement de données (13) est configuré pour exécuter l'étape suivante :
i) vérifier si chaque deuxième caractère trouvé à l'étape e) est lié à une instruction de préparation.

4. Dispositif selon l'une quelconque des revendications précédentes, dans lequel le dispositif de traitement de données (13) est configuré pour exécuter l'étape suivante :
c') fournir un menu de sélection pour sélectionner l'ingrédient entré dans l'étape a) et / ou pour sélectionner l'instruction de préparation du robot de cuisine (1).

5. Dispositif selon la revendication précédente, dans lequel l'instruction de préparation peut être sélectionnée à partir des paramètres de temps, de vitesse, de température, de pesée et / ou de cuisson à la vapeur.

6. Dispositif selon l'une quelconque des revendications précédentes, dans lequel, à l'étape a), la quantité de l'ingrédient et l'unité de mesure de la quantité sont entrées pour chaque ingrédient.

7. Dispositif selon l'une quelconque des revendications précédentes, dans lequel le dispositif de traitement de données (13) est configuré pour exécuter l'étape suivante :
f') délivrer la séquence de caractères avec représentation des caractères liés.

8. Dispositif selon l'une quelconque des revendications précédentes, dans lequel le dispositif de traitement de données (13) est configuré pour exécuter l'étape suivante :
j) fournir un moyen d'édition pour éditer la séquence de caractères qui a été éditée.

9. Dispositif selon l'une quelconque des revendications précédentes, dans lequel le robot de cuisine (1) comprend une balance, et le microprocesseur (11) est configuré pour commander la balance à l'aide du programme de commande établi.
